# EUROPEAN PATENT SPECIFICATION

(11) **EP 2 841 008 B1**
(45) Date of publication and mention of the grant of the patent: **06.09.2017**
(21) Application number: 13779019.2
(22) Date of filing: 19.04.2013
(51) Int. Cl.: A61F 2/00, A61N 1/05

(54) **IMPLANTABLE MEDICAL DEVICE**
IMPLANTIERBARE MEDIZINISCHE VORRICHTUNG
DISPOSITIF MÉDICAL IMPLANTABLE

(30) Priority: 20.04.2012 DK 201200278
(43) Date of publication of application: 04.03.2015
(73) Proprietor: Neurodan A/S, 9200 Aalborg SV (DK)
(72) Inventor: FJORBACK, Morten, 9260 Gistrup (DK); BORUP, Thomas, 9530 Støvring (DK)
(86) International application number: PCT/DK2013/050116
(87) International publication number: WO 2013/156038

(56) References cited:
- WO-A1-03/028804
- WO-A1-2005/084586
- WO-A1-2010/130528
- DE-A1-102008 054 403
- US-A- 4 784 161
- US-A- 5 865 843
- US-A1- 2009 093 879
- US-B1- 6 558 422
- H.G CRAIGHEAD ET AL: "Chemical and topographical patterning for directed cell attachment", CURRENT OPINION IN SOLID STATE AND MATERIALS SCIENCE, vol. 5, no. 2-3, 1 April 2001 (2001-04-01) , pages 177-184, XP055239793, GB ISSN: 1359-0286, DOI: 10.1016/S1359-0286(01)00005-5

## Description

### Technical field

The present invention generally concerns implantable medical devices. More specifically medical devices intended for stimulation of excitable tissue.

### Background

The nerve-electrode interface is the defining factor for a successful neurostimulation product. Neurostimulation electrodes rely on anchoring in tissue in close proximity of the excitable tissue of interest. The clinical effect relies on a constant distance between the electrode and the tissue to be stimulated.

Trends in the neurostimulation market point towards miniaturization of implantable neurostimulation devices using minimally invasive surgical procedures. Ideally, implantation should be done in the doctor's office without the use of general anesthetics and x-ray guidance. The surgical procedure should be optimized to reduce tissue trauma, infection risk, and cost. Correct placement of the electrode is typically confirmed using electrical stimulation of the target excitable tissue. Electrical stimulation may evoke reflex responses to confirm correct placement or insertion may be guided by the patient's perception of the stimuli.

Normally, permanent neurostimulation electrodes are mechanically anchored in tissue using protruding electrode elements such as tines, bristles, barbs, or threads. US8036756 from Medtronic shows an example of such tissue anchoring means. However, the solution requires an implantation tract that is large enough to comprise the protruding elements leading to increased tissue trauma during implantation. When deployed, the protruding elements make it difficult to relocate the electrode to adjust placement. During explantation, the protruding elements increase tissue trauma and may break off when fully removing a perhaps infected device. While implanted, the tines may cause chronic inflammation due to mechanical pull forces exerted on the lead. This is especially the case when the electrode is implanted in adipose tissue where the mechanical mismatch between the tine elements and the surrounding tissue is large.

One way of providing a better adaption of a device into the human body is by treating the device in such a way as to modify the surface structure to promote tissue anchoring by tissue ingrowth. WO201013052BA1 Swiss Micro Laser GmbH teaches that providing a surface structure with protrusions with a height that lies within a range of 1-15 microns and a diameter in the range of 0.1 to 10 microns can facilitate the ingrowth of a bone implant into bone tissue. However, ingrowth of a device into adipose tissue cannot be facilitated by this provision because of the very different nature of adipose tissue and bone tissue, adipose tissue being soft and fragile and bone tissue hard and rigid. The process that builds and promotes tissue growth on bone tissue is different to the process that seeks to promote tissue growth and anchoring in adipose tissue. Thus the teaching in this document does not solve the problem of affixing a device into adipose tissue.

In addition, DE 10 2008 054403 A1 Robert Bosch GMBH, suggests the process of laser treatment of a sensor surface to provide a surface having micro and nano structures.

US 2009/093879 A1 Debra Wawro, suggests the application of micro- and nano- sized grooves as a surface that facilitates ingrowth for cocklear implants.

Further H.G, Craighead et al, explains in the publication XP055239793, with the title: "Chemical and topographical patterning for directed cell attachment", that a surface with a micro structure of pillars can enhance ingrowth into tissue.

However, taking the cited prior art into account and seeking a solution to anchoring a device in adipose tissue, it seems like there is a need for an improvement of the electrode in order to secure the electrode in a fixed position relative to the nerve of interest over the lifetime of the electrode device without the above mentioned drawbacks.

### Summary of the invention

It is an object of embodiments of the present invention to provide an implantable device which in a better way adapts to the human or animal body in order to overcome or at least reduce the above mentioned disadvantages.

It is a further objective of the invention to provide a system for electrical stimulation of nerves featuring implantable parts which in a better way adapts to the human or animal body for the reasons mentioned above. In the following any example and embodiment which does not fall under the scope of the independent claims is not part of the invention. Investigations have shown that providing implantable devices that in a better way adapts to the human or animal body can be achieved if the ingrowth of the implantable device can be accelerated ensuring a quick fixation of the implanted electrode in the animal or human tissue. Different physical surface topographies can accelerate the nucleation and growth and thereby accelerate the number of adherent cells and can therefore affect adhesion of both neural and non-neuronal cell types.

The biological response to an implant can to some extent be controlled through protein coating of the material using, e.g. fibronectin or collagen. However, the effect may only be temporary and regulatory requirements for combination products (device and drug) are higher due to the increased risk for local or systemic adverse reactions. This introduces increased costs for the manufacturer.

The present invention is based on topographical surface treatment of the surface of the electrode using microstructures that promote cell adhesion. The physical micro patterning of the involved implant parts is used to provide topographic stimuli to cells for obtaining good cell adhesion. This can be combined with methods to improve surface wettability by e.g. plasma treatment. In an embodiment, indentations in the lead body can further improve tissue anchoring.

The technology is based on physically transferring the micropattern from the mould to the implant parts or alternatively by hot embossing the micropattern into the part. The microstructures can be applied to the surface of injection moulding tools to facilitate permanent structuring of polymer surfaces during mass production. The easy-release properties of the microstructured mould can be further improved by using e.g. a CrN coating.

The implantable device is intended for chronic implantation and should accordingly be produced of biocompatible materials. Preferred materials are ceramics, metals or polymers depending on the specific use of the implantable device. For neurostimulation devices, it will be preferred to use dielectric biomaterials such as PEEK, PDMS, PU, ETFE, PTFE for electrode bodies, lead jackets, housings and fixation elements.

The low surface energy and hydrophobic nature of the commonly used biocompatible polymers for medical implants, such as PDMS, inhibit cell adhesion. Topographical patterning of the polymer with or without chemical surface modifications such as reactive ion etching (e.g. plasma oxidation) changes the biological response and permits cell adhesion due to improved wettability.

The implantable device is adapted for implantation in the human or animal body and can be adapted especially for a specific purpose as e.g. required for a neurostimulation device, the device including tissue anchoring means for fixation in tissue where the tissue anchoring means are based on a permanent topographical patterning on at least an area of a surface of the device.

For better mechanical interlocking with tissue and providing quick fixation in tissue, the device can in a further embodiment be equipped with indentations with a width in the range from 100-5000 µm and a depth of the indentations in the range between 50-500 µm. The topographical patterning can be applied on all surfaces since the measure of the indentations is far larger than the topographical patterns. Furthermore, the indentations can be carried out in all directions of the surface of the implantable device. Thus the indentations can both be in the longitudinal direction and/or in the axial direction or formed as a worm that travels around the shape of the device in a helical fashion. In other words the indentations can be in any shape on the device that provides a reliable fixation in the tissue.

In an appreciated embodiment, the permanent topographical patterning is imprinted physically by the injection mould or alternatively by hot embossing. More specifically the topographical patterning is formed as structures in the form of pillars/islands protruding out of the surface or wells/pits into the surface. For securing the effect of facilitating fast ingrowths of the device into tissue, the width of the structures is in the range from 1 to 10 µm with a height/depth of 1 to 50 µm, and edge-to-edge spacing between 1 to 10 µm.

The structures are distributed over the surface of the implantable device in either a regular or random pattern or in a combination of regular and random patterns.

In an appreciated embodiment the device is an electrode where a cross section of the electrode body is less than 2 mm and the total length of the electrode body is less than 25 mm. To reduce the risk of rotation, the electrode body can have an arbitrary rounded polygon shape with planar surfaces which also facilitates the application of microstructures. The diameter of the electrode body is larger than the lead wire. When the lead wire gets encapsulated in fibrous tissue this will further assist in anchoring of the device.

It is appreciated that the implantable device can be part of a system for electrical stimulation of nerves. This could be the case if the implantable device is a pulse generator, a lead or an electrode. Further implantable devices like tines, wings, bristles, barbs or threads (tissue anchors) if needed for mechanically securing the device in tissue, can be equipped with the advantageous feature for securing a fast ingrowth in tissue.

The stimulation electrode comprises at least one electrode contact comprising at least one of the following metals: Pt, Ir, Ta, Ti or alloys hereof. The electrode contact surface can further be coated to increase the electrochemical surface area by adding a layer of one of the following thin film coatings: TiNₓ, IrOₓ, Pt, ZrN. A coating layer comprising at least one of the following doped or non-doped carbon-based materials: nanocrystalline diamond, diamond-like carbon, or glassy carbon will improve the electro chemical performance and biocompatibility. The coating layer comprising carbon-based materials as mentioned can be applied directly to the metallic electrode substrate or as a layer on top of another thin film coating layer.

Correct placement of the stimulation electrode is vital for obtaining the desired clinical outcome. Commercially available medical leads are typically implanted using seldinger technique similar to that of installing a central venous line. It is a multistep procedure with several components such as concentric needles, guide wires, dilators and introducers. The nerve to be stimulated is located using the concentric needle and a guide wire is put in place to guide an introducer that will dilate the implantation tract. The medical lead can then be introduced and test stimulation can be used to confirm correct placement.

The present invention facilitates a simple implantation procedure of the electrode because of the unique non-protruding tissue anchoring means where the permanent electrode can be used to stimulate tissue during insertion. It is advantageous to stimulate with the permanent electrode contact during insertion because it eliminates several steps of the normal procedure. Hence, dislocation of needles and guide wires is of no concern.

Especially appreciated is a tool adapted for receiving the electrode device where the implantation tool is comprising an introducer sheath in the form of a tube, the tube having a longitudinal slit for receiving the lead wire and guiding means for holding the electrode body in such a way that the electrode tip is exposed to serve as a blunt element for piercing through tissue and concurrently allow electrical stimulation during insertion. The implantation tool is further comprising a handling arrangement that allows for disengaging the electrode when in place and retracting the implantation tool.
Since the electrode does not rely on protruding electrode elements, the diameter of the implantation tool can be very small and hence will facilitate simple implantation and explantation procedures in accordance with market requirements.

The lack of protruding tissue anchors is also advantageous in case it is desired to explant the electrode due to e.g. infection, pain or lack of clinical efficacy.

Especially appreciated is an explantation tool adapted for removing the electrode device from tissue where the explantation tool comprises a first tubular formed bladed cannula adapted for embracing the lead wire and advancing trough tissue by cutting until reaching a stop formed by the electrode body. The explantation tool further comprises a second tubular cannula adapted for embracing the first cannula and further advancing to cut free the electrode body until a second stop is reached, the distance between the first and the second stop being defined by the length of the electrode body. Thus it is assured that the second cannula is not advanced longer than to cut free the electrode body. This has the advantage that the nerve of interest is protected against damage. The second tubular cannula is further being adapted to support the electrode device during retraction and thus the electrode device can in a simple and minimal invasive operation be explanted.

In one embodiment, the short-term tissue anchoring is reinforced using a mechanical tissue anchor with protruding elements that is slid along the lead after electrode implantation to prevent electrode migration due to lead pulling. The short-term tissue anchor is only intended for temporary reinforcement and could be bioabsorbable. The fixation device is believed to be particularly well anchored because it is implanted through a tract with smaller diameter than the fixation means. The fixation means are protruding elements such as tines, wings, bristles, or barbs.

It will be appreciated that the implantable devices described in the application and optionally the tools for inserting and removing the electrode device can be considered as a kit for an implantable system for electrical nerve stimulation.

In an application of the system for the treatment of incontinence it is appreciated that the at least one electrode is implantable at a left and/or right genital nerve or in the pudendal canal via the pelvic floor.

### Brief summary

In general the invention concerns a surface treatment of an implantable medical device that facilitates tissue anchoring in the human or animal body.

The invention comprises a lead with at least one distal electrode contact and a non-protruding fixation element at the distal end close to the electrode contact(s). The proximal end of the lead can be connected to a pulse generator. Furthermore, the invention comprises an implantation and explantation tool that is adapted for electrode placement in soft tissue.

One of the main concerns is that the electrode will migrate away from the nerve over time causing lack of clinical efficacy. In the present invention, the electrode is constructed in such a way that the cells of the body will adhere to the surface of the electrode and hold it in place utilizing the normal foreign body reaction. This can be considered a "bioactive" anchoring method where the microstructured implant surface provides topographical stimuli to cells. The microstructures are applied to the electrode body by microstructuring the injection mould or by hot embossing. This provides an economically attractive way to mass produce the micron-scale features.

### Description of the drawing

Fig. 1 illustrates the distal part of the electrode lead system consists of an electrode contact, a microstructured electrode body with indentations, and a lead wire,
Fig. 2 illustrates an axonometric projection of the implantable electrode consisting of an electrode contact, electrode body, and lead,
Fig. 3 illustrates a non-cylindrical electrode body with planar surfaces on the sides,
Fig. 4 illustrates the electrode loaded into an implantation tool. The electrode is held in place in the introducer sheath by a tube with a longitudinal slit. The introducer and inner tube has handles to allow retracting the introducer sheath when the electrode is in place,
Fig. 5 illustrates the principle of enhanced short-term tissue anchoring by sliding a mechanical tissue anchor along the lead after electrode implantation to prevent electrode migration due to lead pulling. The fixation device is believed to be particularly well anchored because it is implanted through a tract with smaller diameter than the fixation means. The fixation means are protruding elements such as tines, wings, bristles, or barbs,
Fig. 6 illustrates an axonometric projection of a surface topography consisting of micron sized islands/pillars. A top and side view is also shown,
Fig. 7 illustrates an indentation in the electrode body. Microstructures are present in both the electrode surface and in the recess. Shortly after implantation of the device, cells will migrate into the recess and help prevent dislodgement. The normal foreign body reaction will cause fibrous encapsulation and cell adhesion in the recess further improving tissue integration and anchoring,
Fig. 8 shows an embodiment of the introducer where the cannula has a sharp edge to ease advancement through e.g. connective tissue,
Fig. 9 shows an embodiment of the explantation tool. A bladed cannula is used to cut open the fibrous encapsulation around the lead wire while it is advanced along the lead wire. When the cutting tool has reached the electrode body, a explantation sheath with sharp edges can be advanced through the tissue until it reaches the electrode tip. The electrode can then be removed by pulling on the lead wire. The explantation tool is designed in such a way that it is not possible to cut further than the electrode tip, and
Fig. 10 shows a Scanning Electron Microscope (SEM) image of a thin film surface coating of the electrode contact to improve electrochemical surface area.

### Detailed description

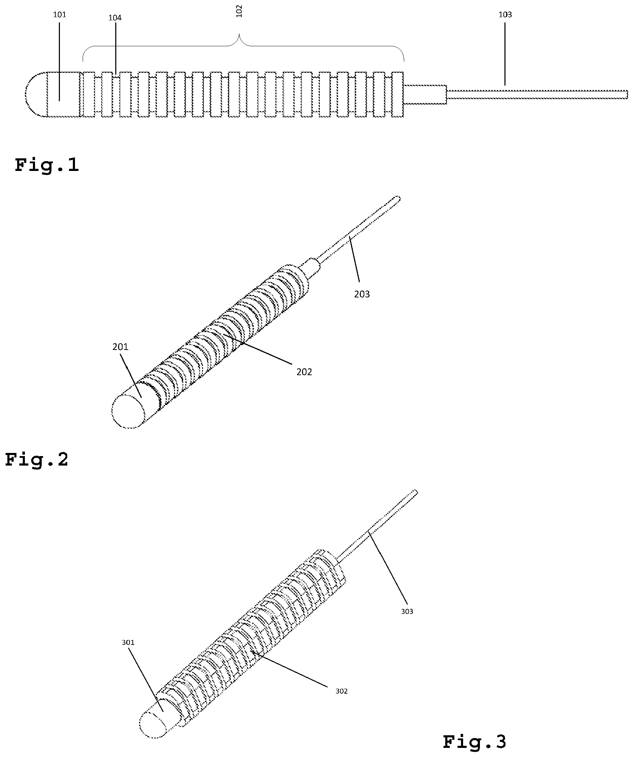

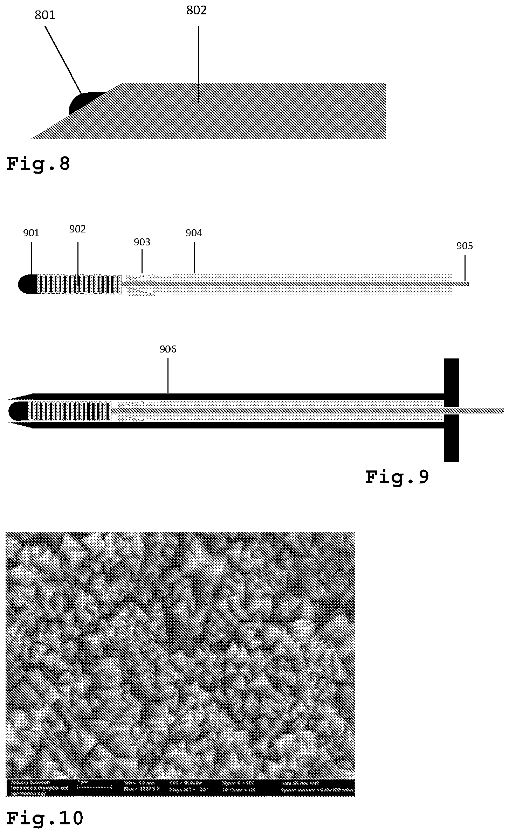

The electrode consists of an electrode contact(s) 101,201, 301, an electrode body 102, and a lead 103, 203, 303. The geometrical surface area of the electrode contact is between 2 and 20 mm2 and has a rounded shape with no sharp edges. A porous coating and/or substrate are used to increase the electrochemical surface area hereby increasing charge injection capacity and reducing electrical impedance of the electrode. The proximal end of the lead 103 can be connected to a pulse generator or other devices via a connector. The electrode contact (s) 101, 201, 301 is the electrochemically active area of the electrode where charge transfer occurs during stimulation. The electrode in fig. 1-3 has a monopolar configuration but additional contacts can be added on demand. The electrode contact is supposed to be in close proximity of the target nerve to obtain low stimulation thresholds. Ideally, the electrode contact(s) should have good chemical stability, high charge injection capacity, low electrical impedance, and should be fully integrated in the tissue as a compliant material causing low degree of inflammation. Reduction of the physical size of the electrode contact will reduce tissue trauma and scarring from insertion and diminish the inflammatory response. However, miniaturization of electrodes is limited by the charge storage capacity and impedance of currently applied materials. The clinical success of electrical stimulation-based systems depends among other things on the ability of the electrode contact to chronically provide safe levels of therapeutic stimulation to a target component of the nervous system. Exceeding the limit for safe charge injection may cause electrode degradation and/or irreversible tissue damage resulting in loss of clinical efficacy. To mitigate the problems associated with reduced physical size, advanced biomaterials and nanocoatings will be used to ensure long-term viability. Typically, stimulation electrode contacts are made of metals such as Pt, Ir, Ta, Ti and alloys hereof. The electrode contact surface 101 can be coated to increase the electrochemical surface area by e.g. thin film deposition of TiNₓ, TrOₓ, Pt, ZrN on a substrate (e.g.: Pt/Ir,Ti or stainless steel alloy types). A Scanning Electron Microscope (SEM) image of such a porous coating is shown in figure 10. Another alternative is carbon-based coatings, such as nanocrystalline diamond, diamond-like carbon, or glassy carbon. These may be doped to further increase their electrochemical and mechanical performance. Sputter deposition is a good method to apply the thin film coating that allows tuning the crystal structure, morphology and chemical composition of the coating by varying several parameters during deposition. To increase the electrochemical surface area, it can be advantageous to use an electrode substrate of e.g. porous titanium created by e.g. titanium sintering, moulding, foaming, or etching. Porous titanium has been used for orthopedic implants as a bone substitute material. Chemical vapor deposition methods are particularly useful when porous substrates are used since sputter deposition is a line-of-sight technique. An example of a suitable coating for a porous substrate could be heavily boron doped nanocrystalline diamond created by microwave assisted CVD which causes reduced inflammation because of the excellent biocompatibility. Electrode implantation results in a foreign body reaction causing fibrous encapsulation of the electrode that increases electrical impedance. This response also increases with micromotions of the electrode with respect to the surrounding tissue which is clearly unwanted. Another option is to combine highly porous coatings such as N-rich TiN with a carbon based layer to improve biocompatibility.

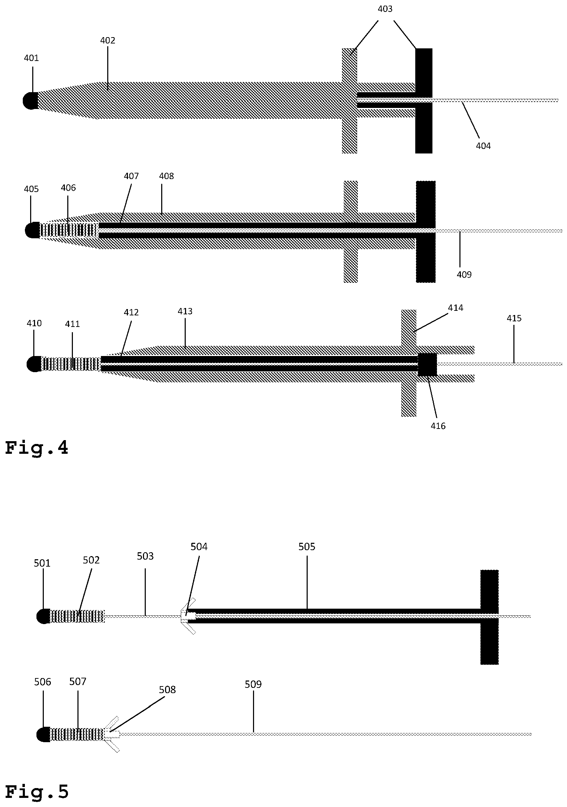

When used for tunneling, the electrode contact 401, 405, 410 is preferably bullet shaped with no sharp edges that can cut tissue or result in local high current density during stimulation. The bullet shape of fig. 1-5 was found especially suitable for implantation into adipose tissue. During insertion, it is possible to stimulate and use a reflex or motor response for guidance. This reveals when the electrode is in the optimal position with the lowest possible activation threshold.

For explanation of the invention a system for treatment of urinary incontinence is used. Thus in the specific embodiment the electrode is adapted and configured for implantation in close proximity of the genital nerves to treat pelvic disorders. The human studies performed so far have shown that the electrode can be placed in a matter of minutes using palpation of the anatomical structures in the region during local anaesthesia. It has proven advantageous to guide the insertion according to the patient's perception of the stimulation together with evocation of the genito-anal reflex to ensure correct placement.

In the preferred embodiment, the physical size of the electrode is adapted for implantation in close proximity of a peripheral nerve in soft tissue for the treatment of pelvic disorders. Anatomical studies in human cadavers and patients have suggested that an electrode body 102 length of less than 25 mm and a diameter of less than 1.2 mm are especially suitable.

Implantation of a medical device into the body evokes the foreign body response. Although a device is considered biocompatible, the body will try to isolate the device from the rest of the body by fibrous encapsulation. The fibroblast is the main cell type involved in formation of the fibrous capsule surrounding an implant in soft tissue. Normally, it will not adhere to the surface and as a consequence, a space, called dead space, will be present between the capsule and the implant. In this capsule the device will be able to move and cause mechanical irritation, which may lead to chronic inflammation. Movement of the implant may promote accumulation of serous fluid at the tissue-implant interface leading to significant clinical problems. The accumulated fluid may cause a low-resistance path between the electrical contacts, which reduces the performance of the device. To avoid this series of problems associated with implantation of medical devices, it is suggested that microstructuring of the implant surface will lead to adhesion of e.g. fibroblasts, thereby eliminating micromotions and the subsequent complications.

Material characteristics, geometry and dimensions as well as surface texture are important to achieve the desired functionality and manufacturability of the electrode. The electrode body comprises in the preferred embodiment a biocompatible dielectric polymer such as PEEK, PDMS, ETFE, or PU. A permanent topographical structuring of the electrode body will be applied to increase cell adhesion and to affect the immunological response to the implant. It may be an advantage if the electrode body is radiopaque due to material selection or an additive. The electrode can then be located using x-ray based imaging modalities.

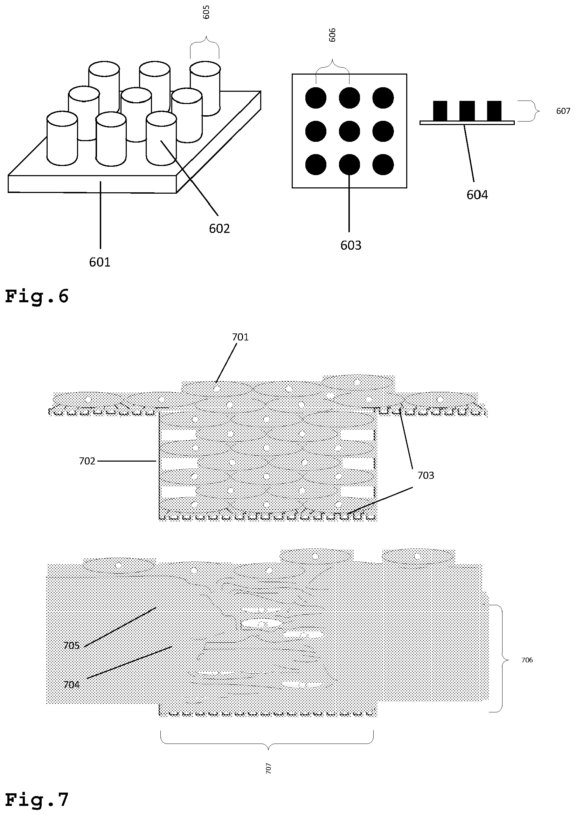

Micro injection moulding has recently emerged as a viable manufacturing route for polymer, metal and ceramic components with micro-scale features and surface textures. The process offers the capability for mass production of microscale devices at low marginal cost. The micro moulding process is typically performed using either modified conventional injection moulding machines, or bespoke machines optimized for the manufacture of micro components. Such machines usually use a dosing piston to inject a tightly controlled amount of polymer into the mould cavities at high velocity. Replication of the small scale features may be improved by applying the so-called injection-compression moulding process applied to micro components.
The micro structures can be islands or pillars distributed over the surface of the implantable device in a regular 601, 603, 604 or random pattern as shown in figure 6. Experimentation in animals has revealed surprisingly good tissue adhesion with a micro feature size 605 of 1-10 microns spaced 1-10 microns apart 606 with a height 607 of 1-50 microns. Together with ion etching of the surface to make the surface hydrophilic, these features were found to cause excellent tissue adhesion that will eliminate movement of the implant. Additional in vitro testing of micro structured surfaces revealed that fibroblasts adhere directly on the implant surface.

The electrode body 102 may further comprise a number of indentations 104, 702 in the surface of the electrode body to further improve tissue anchoring. Edges 705 are rounded to reduce the risk of tissue trauma. Microstructures are present in both the electrode surface and in the recess 703. Shortly after implantation of the device, cells 701 will fill the recess and help prevent dislodgement. The normal foreign body reaction will cause fibrous encapsulation 704 and cell adhesion in the recess 702, further improving tissue integration and anchoring. Longitudinally or axially arranged indentation 104 in the implant surface were found to be very effective for tissue anchoring in adipose tissue with a depth 706 of 50 to 500 micrometers. The preferred recess width with axially arranged indentations 707 is from 100 to 5000 micrometers.

To reduce the risk of rotation, the electrode body can have an arbitrary rounded polygon shape as shown in figure 3. From a production point of view it is easier to apply microstructures to the planar surfaces 302 while de-moulding becomes easier.

The electrode lead 103, 203 must offer high electrical conductivity and be resistant to metal fatigue and corrosion. Additionally, it should be mechanically flexible yet have sufficient break load to allow explantation where pulling on the lead can be expected. Materials suitable for this purpose are coils or strands of high performance alloys such as Pt/Ir or MP35NLT (CoNiCrMo) coated with a dielectric layer of e.g. a fluoropolymer, parylene or PDMS. In case of multiple electrode contacts, a multistranded coil is used. Ideally, the lead is mechanically attached to the electrode contact 101 by crimping or alternatively by welding. The diameter of the electrode body 102 is significantly larger than the lead wire 103. When the lead wire gets encapsulated in fibrous tissue this will further assist in anchoring of the device.

Since the electrode has no protruding fixation elements, a simple implantation and explantation procedure can be applied. The electrode 406 can be loaded into an implantation tool where the electrode is held in place in the introducer sheath 408 by a tube 407 with a longitudinal slit. The introducer and inner tube has handles 403 to allow retracting the introducer sheath when the electrode is in place 414, 416. The longitudinal slit or peel-away design 412 of the introducer will make sure that the lead wire can get out of the tool after implantation. In certain cases it may be advantageous that the introducer cannula 802 has a sharp edge to ease advancement through e.g. connective tissue as shown in figure 8. The Electrode contact 801 is exposed to allow stimulation during insertion.

An explantation tool based on a bladed cannula 904 can be used to cut open the fibrous encapsulation around the lead wire 905 while it is advanced along it. When the cutting tool 903 has reached the electrode body 902, an explantation sheath 906 with sharp edges can be advanced through the tissue until it reaches the electrode tip. The electrode can then be removed by pulling on the lead wire. The explantation tool is designed in such a way that it is not possible to cut further than the electrode tip.

In one embodiment, the short-term tissue anchoring of the electrode 502 is reinforced using a mechanical tissue anchor 504 with protruding elements that is slid along the lead 503 after electrode implantation using 505 to prevent electrode migration due to lead pulling. The short-term tissue anchor is only intended for temporary reinforcement and could be bioabsorbable. The fixation device is believed to be particularly well anchored because it is implanted through a tract with smaller diameter than the fixation means. The fixation means are protruding elements such as tines, wings, bristles, or barbs.

Even though the invention is explained using a specific embodiment that targets a system for the treatment of urinary incontinence, it will be appreciated that the application is not limited by this embodiment but covers all implantable devices, being active or passive, intended for neurostimulation, prosthetics or for plastic surgery where the invention solves the technical problem of providing a device with technical features that facilitate fast ingrowth of the implant in biological tissue.

## Claims

1. An implantable device adapted for implantation in the human or animal body, the device including tissue anchoring means for fixation in tissue where the tissue anchoring means comprises a permanent topographical patterning on at least an area of a surface of the device
**characterized in,**
**that** the device is further:
- equipped with indentations where the dimensions of the indentations is having a width in the range from 100-5000 µm and a depth in the range between 50-500 µm and where
- the topographical patterning is formed as structures in the form of pillars/islands protruding out of the surface of the indentation or wells/pits into the surface of the indentation and where the width of the structures is in the range from 1 to 10 µm with a height/depth of 1 to 50 µm, and edge-to-edge spacing between 1 to 10 µm and where
- the permanent topographical patterning is imprinted physically by an injection mould, alternatively by hot embossing.

2. Implantable device according to claim 1, **characterized in,**
**that** the indentations are formed in the longitudinal direction and/or in the axial direction or formed as a worm that travels around the shape of the device in a helical fashion.

3. Implantable device according to claim 1, **characterized in,**
**that** the device is at least one of the following: an electrode, a pulse generator, a lead, a tissue anchor.

4. Implantable device according to claim 3, **characterized in,**
**that** a cross section of the electrode body is less than 2 mm and the total length of the electrode body is less than 25 mm.

5. Implantable device according to claim 3, **characterized in,**
**that** the electrode body has an arbitrary rounded polygon shape with planar surfaces facilitating the application of microstructures.

6. Implantable device according to claim 3, **characterized in,**
**that** the diameter of the electrode body is larger than the lead wire.

7. Implantable device according to claim 3, **characterized in,**
**that** the electrode is a stimulation electrode and comprises at least one electrode contact, said at least one contact comprising at least one of the following metals: Pt, Ir, Ta, Ti or alloys hereof and the electrode contact surface is coated to increase the electrochemical surface area comprising at least one of the following thin film coatings: TiNₓ, IrOₓ, Pt, ZrN and/or at least one of the following doped or non-doped carbon-based coatings: nanocrystalline diamond, diamond-like carbon, or glassy carbon.

8. Implantable device according to claim 3, **characterized in,**
**that** the electrode body comprises a dielectric material in form of at least one of the following polymers: PEEK, PDMS, PU, ETFE, PTFE the polymer optionally comprising a radiopaque additive and/or being biocompatible.

9. Implantable device according to claim 1, **characterized in,**
**that** the implantable device comprises tines, wings, bristles, barbs or threads for mechanically securing the device in tissue.

10. Implantable device according to any of the preceding claims,
**characterized in,**
**that** the structures are distributed over the surface of the implantable device in either a regular or random pattern or in a combination of regular and random patterns.

11. Implantable device according to any of the preceding claims,
**characterized in,**
**that** the surface of the device is further modified using e.g. plasma oxidation to increase surface wettability.

12. Implantable device according to any of the preceding claims,
**characterized in,**
**that** the device is formed as a mechanical tissue anchor with protruding elements in form of at least one of tines, wings, bristles or barbs, the mechanical tissue anchor having a feed through hole for adapting the mechanical tissue anchor to be slid along the lead wire of the electrode device until a stop is formed by the electrode body, the device optionally being formed by a bioabsorbable material.

13. A system according to any of claims 3 to 12,
**characterized in,**
**that** the at least one electrode is implantable at a left and/or right genital nerve or in the pudendal canal via the pelvic floor.

## Patentansprüche

1. Eine implantierbare Vorrichtung, die für eine Implantation im menschlichen oder tierischen Körper geeignet ist, wobei die Vorrichtung eine Gewebeverankerungseinrichtung zur Fixierung im Gewebe aufweist, wobei die Gewebeverankerungseinrichtung eine permanente topographische Strukturierung auf mindestens einem Oberflächenbereich der Vorrichtung aufweist, **dadurch gekennzeichnet, dass** die Vorrichtung weiter:
- mit Vertiefungen versehen ist, wobei die Abmessungen der Vertiefungen eine Breite im Bereich von 100-5.000 µm und eine Tiefe im Bereich zwischen 50-500 µm haben und wobei
- die topographische Strukturierung als Strukturen in Form von Kissen/Inseln, die aus der Oberfläche der Vertiefungen vorstehen, oder als Absenkungen/Eindrückungen in die Oberfläche der Vertiefungen ausgebildet sind und wobei die Breite der Strukturen im Bereich von 1-10 µm mit einer Höhe/Tiefe von 1-50 µm und einem Rand-zu-Rand-Abstand zwischen 1-10 µm liegt und wobei
- die permanente topographische Strukturierung physikalisch durch ein Spritzgießen aufgedruckt wird, alternativ durch Heißprägen.

2. Implantierbare Vorrichtung nach Anspruch 1, **dadurch gekennzeichnet, dass** die Vertiefungen in Längsrichtung und/oder axialer Richtung ausgebildet sind oder als Schnecke ausgebildet sind, die die Form der Vorrichtung schraubenlinienförmig umgibt.

3. Vorrichtung nach Anspruch 1, **dadurch gekennzeichnet, dass** die Vorrichtung mindestens eine der folgenden ist: einer Elektrode, ein Impulsgenerator, eine Leitung, ein Gewebeanker.

4. Implantierbare Vorrichtung nach Anspruch 3, **dadurch gekennzeichnet, dass** ein Querschnitt des Elektrodenkörpers weniger als 2 mm beträgt und die Gesamtlänge des Elektrodenkörpers weniger als 25 mm beträgt.

5. Implantierbare Vorrichtung nach Anspruch 3, **dadurch gekennzeichnet, dass** der Elektrodenkörper eine beliebige abgerundete Polygonform mit ebenen Flächen aufweist, die das Aufbringen von Mikrostrukturen erleichtern.

6. Implantierbare Vorrichtung nach Anspruch 3, **dadurch gekennzeichnet, dass** der Durchmesser des Elektrodenkörpers größer ist als der Leitungsdraht.

7. Implantierbare Vorrichtung nach Anspruch 3, **dadurch gekennzeichnet, dass** die Elektrode eine Stimmulationselektrode ist und mindestens einen Elektrodenkontakt aufweist, wobei der mindestens eine Kontakt mindestens eines der folgenden Metalle aufweist: Pt, Ir, Ta, Ti oder Legierungen hiervon und die Elektrodenkontaktoberfläche beschichtet ist, um den elektrochemischen Oberflächenbereich zu vergrößern, die mindestens eine der folgenden Dünnfilmbeschichtungen umfasst: TiNₓ, IrOₓ, Pt, ZrN und/oder mindestens eine der folgenden dotierten oder undotierten kohlenstoffbasierten Beschichtungen: nanokristalliner Diamant, diamantartiger Kohlenstoff oder glasartiger Kohlenstoff.

8. Implantierbare Vorrichtung nach Anspruch 3, wobei der Elektrodenkörper ein di-elektrisches Material in Form von einem der folgenden Polymere aufweist: PEEK, PDMS, PU, ETFE, PFTE, wobei das Polymer gegebenenfalls ein strahlungsundurchlässiges Additiv enthält und/oder biokompatibel ist.

9. Implantierbare Vorrichtung nach Anspruch 1, **dadurch gekennzeichnet, dass** die implantierbare Vorrichtung Zinken, Flügel, Borsten, Widerhaken oder Fäden zum mechanischen Befestigen der Vorrichtung im Gewebe umfasst.

10. Implantierbare Vorrichtung nach einem der vorhergehenden Ansprüche, **dadurch gekennzeichnet, dass** die Strukturen über die Oberfläche der implantierbaren Vorrichtung entweder in einem regelmäßigen oder einem zufälligen Muster oder in Kombination von regelmäßigen und zufälligen Mustern verteilt sind.

11. Implantierbare Vorrichtung nach einem der vorhergehenden Ansprüche, **dadurch gekennzeichnet, dass** die Oberfläche der Vorrichtung weiter modifiziert ist unter Verwendung von z. B. Plasmaoxidation, um die Oberflächenbenetzbarkeit zu vergrößern.

12. Implantierbare Vorrichtung nach einem der vorhergehenden Ansprüche, **dadurch gekennzeichnet, dass** die Vorrichtung als ein mechanischer Gewebeanker ausgebildet ist mit vorstehenden Elementen in Form von mindestens einen von Zinken, Flügeln, Borsten oder Widerhaken, wobei der mechanische Gewebeanker mit einem Durchgangsloch versehen ist, um den mechanischen Gewebeanker dazu anzupassen, entlang des Leitungsdrahts der Elektrodeneinrichtung verschoben zu werden, bis ein Anschlag durch den Elektrodenkörper gebildet ist, wobei die Vorrichtung wahlweise durch ein bioabsorbierbares Material gebildet ist.

13. Ein System nach einem der Ansprüche 3 bis 12, **dadurch gekennzeichnet, dass** die mindestens eine Elektrode an einem linken und/oder rechten Nervus Genitales oder in dem Pudendalkanal über dem Beckenboden implantierbar ist.

## Revendications

1. Dispositif implantable destiné à une implantation dans le corps humain ou animal, le dispositif incluant un moyen d'ancrage de tissu pour une fixation dans un tissu dans lequel le moyen d'ancrage de tissu comprend une structuration topographique permanente sur au moins une zone d'une surface du dispositif
**caractérisé en ce,**
**que** le dispositif est en outre :
- équipé d'indentations dans lequel les dimensions des indentations présentent une largeur dans la plage de 100-5000 µm et une profondeur dans la plage allant de 50-500 µm et dans lequel
- la structuration typographique est conçue comme des structures sous la forme de piliers/îlots faisant saillie depuis la surface de l'indentation ou de puits/fosses dans la surface de l'indentation et dans lequel la largeur des structures est dans la plage de 1 à 10 µm avec une hauteur/profondeur de 1 à 50 µm, et un espacement bord à bord entre 1 et 10 µm et dans lequel
- la structuration topographique permanente est physiquement imprimée par un moule à injection, autrement par estampage à chaud.

2. Dispositif implantable selon la revendication 1, **caractérisé en ce,**
**que** les indentations sont formées dans la direction longitudinale et/ou dans la direction axiale ou conçues comme une vis qui se déplace autour de la forme du dispositif de façon hélicoïdale.

3. Dispositif implantable selon la revendication 1, **caractérisé en ce,**
**que** le dispositif est au moins l'un des éléments suivants :
une électrode, un générateur d'impulsions, un raccord, un élément d'ancrage de tissu.

4. Dispositif implantable selon la revendication 3, **caractérisé en ce,**
**qu'**une section transversale du corps d'électrode est inférieure à 2 mm et la longueur totale du corps d'électrode est inférieure à 25 mm.

5. Dispositif implantable selon la revendication 3, **caractérisé en ce,**
**que** le corps d'électrode présente une forme de polygone arrondi quelconque dotée de surfaces planes facilitant l'application de microstructures.

6. Dispositif implantable selon la revendication 3, **caractérisé en ce,**
**que** le diamètre du corps d'électrode est plus important que le fil de raccord.

7. Dispositif implantable selon la revendication 3, **caractérisé en ce,**
**que** l'électrode est une électrode de stimulation et comprend au moins un contact d'électrode, ledit au moins un contact comprenant au moins l'un des métaux suivants :
Pt, Ir, Ta, Ti ou des alliages de ceux-ci et la surface de contact d'électrode est revêtue pour augmenter la surface électrochimique comprenant au moins l'un des revêtements en couche mince suivants : TiNₓ, IrOₓ, Pt, ZrN et/ou au moins l'un des revêtements à base de carbone dopés ou non-dopés suivants : du diamant nanocristallin, du carbone de type diamant, ou du carbone vitreux.

8. Dispositif implantable selon la revendication 3, **caractérisé en ce,**
**que** le corps d'électrode comprend un matériau diélectrique sous forme d'au moins l'un des polymères suivants : PEEK, PDMS, PU, ETFE, PTFE le polymère comprenant éventuellement un additif radio-opaque et/ou étant biocompatible.

9. Dispositif implantable selon la revendication 1, **caractérisé en ce,**
**que** le dispositif implantable comprend des dents, des ailettes, des poils, des barbelures ou des filets pour une fixation mécanique du dispositif dans un tissu.

10. Dispositif implantable selon l'une quelconque des revendications précédentes,
**caractérisé en ce,**
**que** les structures sont réparties sur la surface du dispositif implantable soit selon un schéma régulier ou aléatoire soit selon une combinaison de schémas réguliers et aléatoires.

11. Dispositif implantable selon l'une quelconque des revendications précédentes,
**caractérisé en ce,**
**que** la surface du dispositif est en outre modifiée en utilisant par ex. une oxydation par plasma pour augmenter la mouillabilité de surface.

12. Dispositif implantable selon l'une quelconque des revendications précédentes,
**caractérisé en ce,**
**que** le dispositif est conçu comme un élément d'ancrage de tissu mécanique doté d'éléments faisant saillie sous forme d'au moins l'un de dents, d'ailettes, de poils ou de barbelures, l'élément d'ancrage de tissu mécanique présentant un trou traversant d'alimentation pour adapter l'élément d'ancrage de tissu mécanique à glisser le long du fil de raccord du dispositif d'électrode jusqu'à ce qu'une butée soit formée par le corps d'électrode, le dispositif étant éventuellement formé par un matériau bioabsorbable.

13. Système selon l'une quelconque des revendications 3 à 12, **caractérisé en ce,**
**que** l'au moins une électrode est implantable au niveau d'un nerf génital gauche et/ou droit ou dans le canal pudendal via le plancher pelvien.
